# EUROPEAN PATENT SPECIFICATION

(11) **EP 1 279 365 B1**
(45) Date of publication and mention of the grant of the patent: **20.12.2006**
(21) Application number: 02016268.1
(22) Date of filing: 23.07.2002
(51) Int. Cl.: A61B 5/00

(54) **Removable cover for a glucose meter**
Abnehmbarer Schutzdeckel für eine Glukose-Messvorrichtung
Capot protecteur amovible pour un dispositif de mesure de glucose

(30) Priority: 26.07.2001 US 915998
(43) Date of publication of application: 29.01.2003
(73) Proprietor: BAYER CORPORATION, Pittsburgh, PA 15205 (US)
(72) Inventor: Moller, Andreas, 80469 München (DE); Schlagheck, Julian, 81474 München (DE); Steinberg, Claus, 82041 Deisenhofer, München (DE); Dunwald, Christoph, 51373 Leverkusen (DE)
(74) Representative: Linhart, Angela

(56) References cited:
- WO-A-98/17088
- GB-A- 2 333 402
- US-A- 5 714 123
- US-A- 5 745 566
- US-A- 5 772 586

## Description

### FIELD OF THE INVENTION

The present invention relates to a cover for a glucose meter. More particularly, the present invention relates to a cover that can be removably connected with a glucose meter.

### BACKGROUND

Blood glucose meters are used to measure the level of glucose in a user's blood. Generally, to operate a blood glucose meter, a user deposits a drop of blood onto a disposable cartridge or pad. The disposable cartridge along with the drop of blood is then inserted into a slot located on the blood glucose meter whereupon the blood glucose meter tests the blood located on the disposable cartridge in order to determine the level of glucose in the blood. Often times, upon determining the level of glucose in the blood, the blood glucose meter displays this information along with other information on a screen located on the blood glucose meter.

Often times, blood glucose meters are self-contained units having a case or housing manufactured from a rigid material such as plastic. Plastic is prone to becoming scratched and may become cracked or even broken if the blood glucose meter suffers an impact, such as being dropped onto a floor. Additionally, some blood glucose meters houses a screen for displaying information. A screen for displaying information may be very fragile and therefore easily damaged if the blood glucose meter or the screen itself suffers an impact. Moreover, often times the surface of a blood glucose meter may be relatively smooth and lack any features or means for allowing a user to effectively grip the blood glucose meter. If the user is unable to effectively grip the blood glucose meter, the user may lose contact with the blood glucose meter and the blood glucose meter may suffer an impact and become damaged.

Sometimes, blood glucose meters are manufactured using a bland, typically non-descriptive, color. A bland color does not stand out and does not allow the user to easily locate the blood glucose meter if the blood glucose meter is ever lost. Additionally, some blood glucose meters have little or no information located on their surface. If the blood glucose meter has little or no information located on its surface, the blood glucose meter may lack valuable information such as the user's name, instructions on how to use the blood glucose meter, or even a design or pattern which can be used to identify the blood glucose meter.

Accordingly, there is a need for a device that can provide additional gripping means for a user to grip onto the blood glucose meter, which can protect the blood glucose meter from becoming damaged when the blood glucose meter suffers an impact, and which can provide valuable information such as a user's name, instruction's on how to use the blood glucose meter, or a design or pattern which can be used to identify the blood glucose meter.

### SUMMARY

According to the present invention, an apparatus for covering a glucose meter is provided. The glucose meter has a front surface opposed to a back surface, and an edge surface connecting the front surface to the back surface. The apparatus comprises a frame adapted to receive the glucose meter and at least one attachment member connected with the frame. The frame is adapted to surround a substantial portion of the edge surface and form an opening for viewing at least a portion of the front surface of the glucose meter. The attachment member is adapted to removably connect the frame with the glucose meter. An interchangeable faceplate is removably connected with the frame and received by the opening in the frame.

According to one aspect of the present invention the frame is adapted to cover a substantial portion of the front surface and a small portion of the back surface of the glucose meter.

According to another aspect of the present invention, the attachment member contacts a small portion of the back surface.

### BRIEF DESCRIPTION OF THE DRAWINGS

FIG. 1 is a perspective view of a removable cover attached to a glucose meter, in accordance with one embodiment;
FIG. 2 is an exploded perspective view of a removable cover and a glucose meter, in accordance with one embodiment;
FIG. 3 is a front view of a removable cover attached to a glucose meter, in accordance with one embodiment;
FIG. 4 is a rear view of the removable cover of FIG. 3, in accordance with one embodiment;
FIG. 5 is a cross-sectional side view of the removable cover attached to the glucose meter of FIG. 3, in accordance with one embodiment;

For simplicity and clarity of illustration, elements shown in the Figures have not necessarily been drawn to scale. For example, the dimensions of some of the elements are exaggerated relative to each other for clarity. Further, where considered appropriate, reference numerals have been repeated among the Figures to indicate corresponding elements.

### DETAILED DESCRIPTION OF THE PRESENTLY PREFERRED EMBODIMENTS

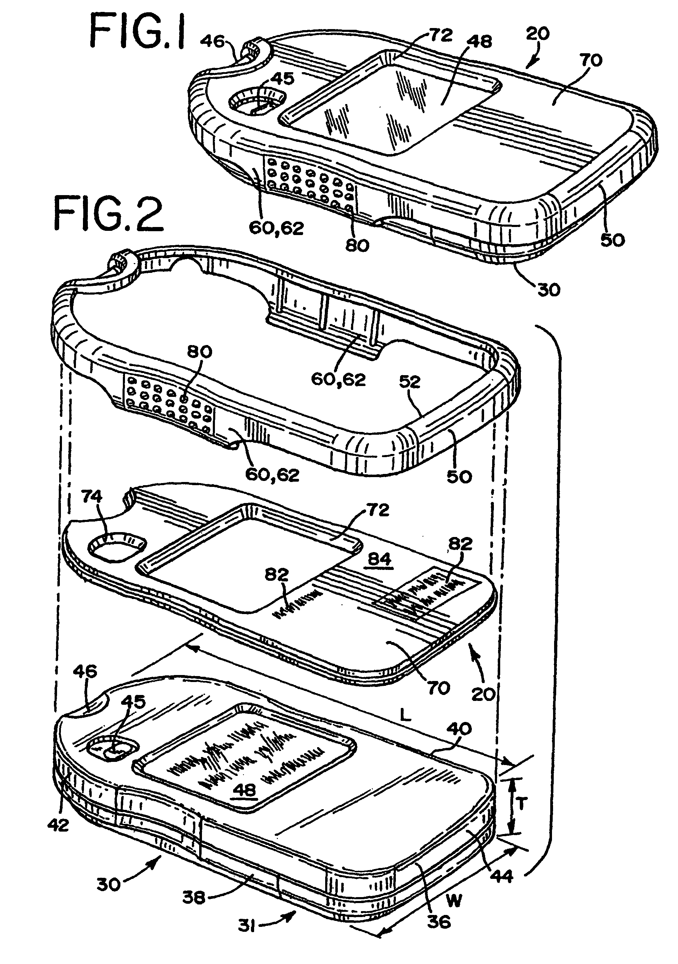

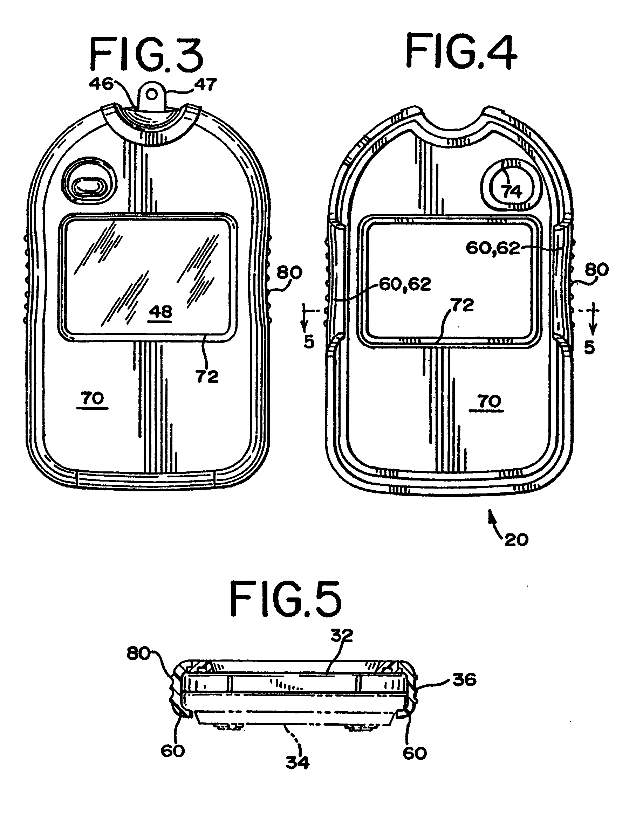

FIGS. 1-5 illustrate one embodiment of a cover 20 for covering a glucose meter 30. Glucose meter 30 can be any device that can be used to determine the level of glucose in a user's blood. Preferably, glucose meter 30 is a portable blood glucose meter that can be carried along with a user, such as the GLUCOMETER ELITE XL^{™}, blood glucose meter available from BAYER CORPORATION^{™} of Territown, New York, United States of America. A portable blood glucose meter has to be light enough so that a user may carry it around. Preferably, a portable blood glucose meter weighs less than 2000 grams, and more preferably, less than 500 grams. A portable blood glucose meter must also have dimensions that are small enough to allow a user to carry it around. Preferably, a portable blood glucose meter has a width W of less than 10 centimeters, a length L of less than 20 centimeters, and a thickness T of less than 5 centimeters, as illustrated in FIG. 2. More preferably, a portable blood glucose meter has a width W of less than 7 centimeters, a length L of less than 12 centimeters, and a thickness T of less than 3 centimeters.

Glucose meter 30 has a front surface 32 opposed to a back surface 34, and .. an edge surface 36 connecting the front surface 32 to the back surface 34, as illustrated in FIGS. 2 and 5. The front surface 32 includes a screen 48 for viewing information, such as the user's blood glucose level. Screen 48 includes any device that can display information such as, but not limited to, a cathode ray tube, an LCD screen, or any other means for allowing a user to view information. Preferably, screen 48 comprises an LCD screen, since an LCD screen is lightweight, thin, and typically compact enough so that a user can easily carry glucose meter 30 around. Preferably front surface 32 also includes a power button 45 for turning the glucose meter 30 on. However, the power button 45 may be located on alternate surfaces of glucose meter 30, such as the back surface 34 or the edge surface 36.

The front surface 32, the back surface 34 and the edge surface 36 form a housing 31 for the glucose meter 30. Preferably, the housing 31 comprises a rigid material, such as, but not limited to: metals such as iron, steel, aluminum, titanium, and brass; plastics such as ethylene-vinyl acetate; acrylics such as acrylonitrile-butadiene-styrene and acrylic-styrene-acrylonitrile; polymers such as polycarbonate, polyurethane, polythylene, polybutylene, polyvinyl chloride, polyphenylene oxide, chlorinated polyvinyl chloride, polyamides, and polybutylene terephthalate; carbon fiber; graphite; and any other rigid material known to those skilled in the art. The housing 31 may be formed in one of many ways known to those skilled in the art, such as die-casting, machine forming, traditional molding, and blow-molding. The housing 31 acts as a means for storing any electronics located within the glucose meter 30 and acts as a means for mounting items such as the screen 48 and the power button 45.

The cover 20 may be colored any color known to those skilled in the art. Preferably, the cover 20 is not colored in a bland color, but rather a distinct color that stands out and allows the user to more easily locate the glucose meter 30 if the glucose meter 30 is ever lost. A distinct color includes such colors as fluorescent colors, metallic colors, bright colors such as yellow, red, orange, intermediate colors such as blue or green, and even solid black or solid white. The cover 20 includes a frame 50 and at least one attachment member 60 connected with the frame 50.

The frame 50 is adapted to receive the glucose meter 30. Preferably, the frame 50 comprises a rigid material, such as, but not limited to: metals such as iron, steel, aluminum, titanium, and brass; plastics such as ethylene-vinyl acetate; acrylics such as acrylonitrile-butadiene-styrene and acrylic-styrene-acrylonitrile; polymers such as polycarbonate, polyurethane, polythylene, polybutylene, polyvinyl chloride, polyphenylene oxide, chlorinated polyvinyl chloride, polyamides, and polybutylene terephthalate; carbon fiber; graphite; and any other rigid material known to those skilled in the art. In one embodiment, the frame 50 is adapted to surround a substantial portion of the edge surface 36. As used herein, a substantial portion of the edge surface 36 is a portion of the edge surface 36 that includes at least 75 percent of the perimeter of the edge surface 36. Preferably a substantial portion of the edge surface 36 is a portion that includes least 90 percent of the perimeter surrounding the edge surface 36, as illustrated in FIGS. 1 and 2. In one embodiment, the frame 50 forms an opening 52 for viewing at least a portion of the front surface 32 of the glucose meter 30, as illustrated in FIGS. 2 and 7. In one embodiment, opening 52 is designed to receive an interchangeable faceplate 70, as illustrated in FIGS. 1 and 2. In one embodiment, opening 52 forms a window 54 for viewing at least a portion of the screen 48, and preferably, for viewing the entire screen 48, as illustrated in FIG. 7.

In one embodiment, the frame 50 does not cover a substantial portion of the back surface 34 of the glucose meter 30. As defined herein, a substantial portion of the back surface 34 of the glucose meter 30 is a portion that includes at least 90 percent of the surface area of back surface 34, and more preferably, that includes at least 50 percent of the back surface 34 of glucose meter 30. In one embodiment, the frame 50 covers a small portion of the back surface 34 of the glucose meter 30. As defined herein, a small portion of the back surface 34 is a portion that includes no more than 20 percent of the service area of back surface 34. By not covering a substantial portion of the back surface 34 or by covering a small portion, the weight of cover 20 is substantially less than it could be, and therefore the combination of the glucose meter 30 and the cover 20 may be relatively light in weight. Moreover, when the frame 50 does not covering a substantial portion of the back surface 34 or covers only a small portion, the frame 50 may be more readily removed from the glucose meter 30. Moreover, a user may more easily switch from one cover 20 to a second cover 20 when the frame 50 does not covering a substantial portion of the back surface 34 or covers only a small portion.

In one embodiment, the frame 50 is adapted to cover a substantial portion of the front surface 32. As defined herein, a substantial portion of the front surface is a portion of the front surface 32 that includes at least 50 percent of the surface area of the front surface 32.

Cover 20 also includes at least one attachment member 60 connected with the frame 50, wherein the attachment member 60 is adapted to removably connect the frame 50 with the glucose meter 30, as illustrated in FIGS. 1 and 6. Attachment member 60 can be generally any type of coupling mechanism known to those skilled in the art, such as, an alligator clip, a clamp, a magnet, a hook and loop type fastener (such as a strip of VELCRO^{™}), a snap fit arrangement or any other comparable coupling mechanism which can be removably coupled to an object, such as the glucose meter 30. In one embodiment, the attachment member 60 is a snap-fit clip 62 that is adapted to removably connect the frame 50 with the glucose meter 30, as illustrated in FIGS. 1 and 2. Attachment member 60 is connected with frame 50 in one of many ways known to those skilled in the art such as, for example, welding, gluing, stapling, snap fit arrangement, a hinge, a nut and bolt arrangement, a screw, or any other means known to those skilled in the art for connecting a first object with a second object. In one embodiment, attachment member 60 is integrally formed with frame 50, as illustrated in FIGS. 1 and 2. In one embodiment, cover 20 comprises a second attachment member 60 connected with the frame 50, wherein the second attachment member 60 is adapted to removably connect the frame 50 with a glucose meter 30, as illustrated in FIG. 2.

The cover 20 includes an interchangeable faceplate 70 which is removably connected with the frame 50 and received by the opening 52 in the frame 50, as illustrated in FIGS. 1 and 2. In this embodiment, opening 52 of frame 50 is adapted to receive the interchangeable faceplate 70, as illustrated in FIG. 2. The interchangeable faceplate 70 is removably connected with the frame 50 using any type of coupling mechanism known to those skilled in the art, such as, an alligator clip, a clamp, a magnet, a hook and loop type fastener (such as a strip of VELCRO^{™}), a snap fit arrangement or any other comparable coupling mechanism which can be removably coupled to an object, such as the frame 50.

In operation, interchangeable faceplate 70 is received by the opening 52 in the frame 50 and then connected with the frame 50. Attachment member 60 is then removably connected with the glucose meter 30, as illustrated in FIGS. 1 and 2. Preferably, the interchangeable faceplate 70 forms a window 72 for viewing the screen 48 located on the front surface 32 of the glucose meter 30.

Interchangeable faceplate 70 may also include information 82 located on the surface 84 of interchangeable faceplate 70. Information 82 includes any information known to those skilled in the art that may be imprinted, embossed, placed, or encoded on the surface 84. Preferably, information 82 includes useful information such as the user's name, instructions on how to use the glucose meter 30, or even a design or pattern which can be used to identify the glucose meter 30.

In one embodiment, glucose meter 30 includes a slot 46 for receiving a disposable cartridge 47, wherein the frame 50 surrounds a portion of the slot 46, as illustrated in FIG. 1 and FIG. 2. In one embodiment, the slot 46 is located on the edge surface 36 of the glucose meter 30, as illustrated in FIGS. 1 and 2. By not covering slot 46, frame 50 of cover 20 allows a user to insert the disposable cartridge 47 into the slot 46 when the frame 50 is connected with the glucose meter 30. In this way, the user may obtain a measurement for the user's blood glucose level using glucose meter 30 without having to remove frame 50.

In one embodiment, edge surface 36 comprises a first side surface 38 opposed to a second side surface 40, and a top surface 42 opposed to a bottom surface 44, as illustrated in FIG. 2 and FIG. 5. In this embodiment, the frame 50 contacts at least a portion of the first side surface 38, the second side surface 40, the top surface 42, and the bottom surface 44. In one embodiment, the frame 50 contacts the edge surface 36 in at least two places, and more preferably in at least three places.

In one embodiment, cover 20 includes a gripping member 80 connected with the frame 50. Gripping member 80 is any type of device known to those skilled in the art that can be used to aid a user in gripping an object, such as, a bump, a ridge, a rubber bump, a rubber ridge, a rubber member, a surface covered with adhering materials, and other such device. Preferably, gripping member 80 is integrally formed with the frame 50, as illustrated in FIGS. 1-4. When cover 20 is connected with glucose meter 30, gripping member 80 helps the user maintain and grip the glucose meter 30.

In one embodiment, a method for distributing disposable cartridges 47 for a glucose meter 30 is described, wherein the method comprises packaging at least one cover 20, as described above, with at least one disposable cartridge 47. By packaging a cover 20 with a disposable cartridge 47, as user is able more easily obtain accessories, such as a cover 20 and a disposable cartridge 47, for a glucose meter 30.

## Claims

1. A glucose meter (30), wherein the glucose meter (30) has a front surface (32) opposed to a back surface (34), and an edge surface (36) connecting the front surface (32) to the back surface (34), the glucose meter (30) comprising:
a screen (48) located on the front surface (32) for viewing information;
a cover (20) removably connected with the glucose meter (30), the cover (20) including a frame (50) adapted to receive the glucose meter (30), wherein the frame (50) is adapted to surround a portion of the edge surface (36), and at least one attachment member (60) connected with the frame (50), wherein the attachment member (60) is adapted to removably connect the frame (50) with the glucose meter (30), and wherein the attachment member (60) contacts a small portion of the back surface (34),and wherein the frame (50) forms an opening (52) for viewing at least a portion of the front surface (32) of the glucose meter (30),
**characterized in that**
the glucose meter (30) further comprises an interchangeable faceplate (70) removably connected with the frame (50) and received by the opening (52) in the frame (50).

2. The glucose meter (30) of Claim 1, wherein the interchangeable faceplate (70) forms a window (72) for viewing said screen (48) located on the front surface (32).

3. The glucose meter (30) of Claim 1 or 2, further comprising a slot (46) for receiving a disposable cartridge (47) located on the edge surface (36), and wherein the frame (50) surrounds the slot (46).

4. The glucose meter (30) of one of the Claims 1 to 3, wherein the frame (50) does not cover a substantial portion of the back surface (34).

5. The glucose meter (30) of one of the Claims 1 to 4, wherein the frame (50) is adapted to cover a substantial portion of the front surface (32) and a small portion of the back surface (34).

6. The glucose meter (30) of Claim 1, wherein the frame (50) forms a window (52) for viewing a screen (48) located on the front surface (32).

7. The glucose meter (30) of one of the Claims 1 to 6 further comprising a gripping member (80) connected with the frame (50).

8. The glucose meter (30) of Claim 7, wherein the gripping member (80) is integrally formed with the frame (50).

9. The glucose meter (30) of one of the Claims 1 to 8, wherein the attachment member (60) is integrally formed with the frame (50).

## Patentansprüche

1. Glucosemessgerät (30), wobei das Glucosemessgerät (30) eine vordere Oberfläche (32), die einer hinteren Oberfläche (34) gegenüberliegt, und eine Kantenoberfläche (36), die die vordere Oberfläche (32) mit der hinteren Oberfläche (34) verbindet, aufweist, wobei das Glucosemessgerät (30) Folgendes umfasst:
einen Schirm (48) zur Betrachtung von Informationen, der auf der vorderen Oberfläche (32) angeordnet ist;
eine Abdeckung (20), die abnehmbar mit dem Glucosemessgerät (30) verbunden ist, wobei die Abdeckung (20) einen Rahmen (50), der ausgebildet ist, um das Glucosemessgerät (30) aufzunehmen, und wobei der Rahmen (50) ausgebildet ist, um einen Abschnitt der Kantenoberfläche (36) zu umgeben, und zumindest ein mit dem Rahmen (50) verbundenes Befestigungselement (60) umfasst, wobei das Befestigungselement (60) ausgebildet ist, um den Rahmen (50) abnehmbar mit dem Glucosemessgerät (30) zu verbinden und worin das Befestigungselement (60) einen kleinen Abschnitt der hinteren Oberfläche (34) kontaktiert und worin der Rahmen (50) eine Öffnung (52) ausbildet, um zumindest einen Abschnitt der vorderen Oberfläche (32) des Glucosemessgeräts (30) zu betrachten,
**dadurch gekennzeichnet, dass**
das Glucosemessgerät (30) ferner eine auswechselbare Vorderplatte (70) umfasst, die abnehmbar mit dem Rahmen (50) verbunden ist und durch die Öffnung (52) im Rahmen (50) aufgenommen ist.

2. Glucosemessgerät (30) nach Anspruch 1, worin die auswechselbare Vorderplatte (70) ein Fenster ausbildet (72), um den Schirm (48), der auf der vorderen Oberfläche (32) angeordnet ist, zu betrachten.

3. Glucosemessgerät (30) nach Anspruch 1 oder 2, das ferner einen Schlitz (46) zur Aufnahme einer Einwegkassette (47), der an der Kantenoberfläche (36) angeordnet ist, umfasst und worin der Rahmen (50) den Schlitz (46) umgibt.

4. Glucosemessgerät (30) nach einem der Ansprüche 1 bis 3, worin der Rahmen (50) keinen wesentlichen Abschnitt der hinteren Oberfläche (34) abdeckt.

5. Glucosemessgerät (30) nach einem der Ansprüche 1 bis 4, worin der Rahmen (50) ausgebildet ist, um einen wesentlichen Abschnitt der vorderen Oberfläche (32) und einen kleinen Abschnitt der hinteren Oberfläche (34) abzudecken.

6. Glucosemessgerät (30) nach Anspruch 1, worin der Rahmen (50) ein Fenster (52) ausbildet, um einen Schirm (48) zu betrachten, der an der vorderen Oberfläche (32) angeordnet ist.

7. Glucosemessgerät (30) nach einem der Ansprüche 1 bis 6, das ferner ein mit dem Rahmen (50) verbundenes Greifelement (80) umfasst.

8. Glucosemessgerät (30) nach Anspruch 7, worin das Greifelement (80) einstückig mit dem Rahmen (50) ausgebildet ist.

9. Glucosemessgerät (30) nach einem der Ansprüche 1 bis 8, worin das Befestigungselement (60) einstückig mit dem Rahmen (50) ausgebildet ist.

## Revendications

1. Glucomètre (30), dans lequel le glucomètre (30) comporte une surface avant (32) à l'opposé d'une surface arrière (34), et une surface de bord (36) reliant la surface avant (32) à la surface arrière (34), le glucomètre (30) comprenant :
un écran (48) situé sur la surface avant (32) pour voir des informations ;
un capot (20) relié de façon amovible au glucomètre (30), le capot (20) comprenant un châssis (50) apte à recevoir le glucomètre (30), le châssis (50) étant apte à entourer une partie de la surface de bord (36), et au moins un élément de fixation (60) relié au châssis (50), l'élément de fixation (60) étant apte à relier de façon amovible le châssis (50) au glucomètre (30), et l'élément de fixation (60) entrant en contact avec une petite partie de la surface arrière (34), et le châssis (50) formant une ouverture (52) pour voir au moins une partie de la surface avant (32) du glucomètre (30),
**caractérisé en ce que**
le glucomètre (30) comprend, en outre, une plaque de façade interchangeable (70) reliée de façon amovible au châssis (50) et reçue par l'ouverture (52) dans le châssis (50).

2. Glucomètre (30) selon la revendication 1, dans lequel la plaque de façade interchangeable (70) forme une fenêtre (72) pour voir ledit écran (48) situé sur la surface avant (32).

3. Glucomètre (30) selon la revendication 1 ou 2, comprenant, en outre, une fente (46) pour recevoir une cartouche jetable (47) située sur la surface de bord (36), et dans lequel le châssis (50) entoure la fente (46).

4. Glucomètre (30) selon l'une des revendications 1 à 3, dans lequel le châssis (50) ne recouvre pas une partie substantielle de la surface arrière (34).

5. Glucomètre (30) selon l'une des revendications 1 à 4, dans lequel le châssis (50) est apte à recouvrir une partie substantielle de la surface avant (32) et une petite partie de la surface arrière (34).

6. Glucomètre (30) selon la revendication 1, dans lequel le châssis (50) forme une fenêtre (52) pour voir un écran (48) situé sur la surface avant (32).

7. Glucomètre (30) selon l'une des revendications 1 à 6, comprenant, en outre, un élément de préhension (80) relié au châssis (50).

8. Glucomètre (30) selon la revendication 7, dans lequel l'élément de préhension (80) est formé d'un seul tenant avec le châssis (50).

9. Glucomètre (30) selon l'une des revendications 1 à 8, dans lequel l'élément de fixation (60) est formé d'un seul tenant avec le châssis (50).
